# EUROPÄISCHE PATENTSCHRIFT

(11) **EP 1 007 934 B1**
(45) Veröffentlichungstag und Bekanntmachung des Hinweises auf die Patenterteilung: **29.01.2003**
(21) Anmeldenummer: 96939881.7
(22) Anmeldetag: 21.11.1996
(51) Int. Cl.: G01N 1/10, G01N 33/18

(54) **VORRICHTUNG ZUR UNTERSUCHUNG VON FLÜSSIGKEITSPROBEN**
DEVICE FOR ANALYZING FLUID SAMPLES
DISPOSITIF POUR ANALYSER DES ECHANTILLONS DE LIQUIDES

(30) Priorität: 01.12.1995 DE 19544851; 03.05.1996 DE 19617707
(43) Veröffentlichungstag der Anmeldung: 14.06.2000
(73) Patentinhaber: ISCO, Inc., Lincoln, Nebraska 68504 (US)
(72) Erfinder: ISCO, Inc., Lincoln, Nebraska 68504 (US)
(74) Vertreter: Katscher, Helmut, Dipl.-Ing.
(86) Internationale Anmeldenummer: EP9605139
(87) Internationale Veröffentlichungsnummer: WO97021088

(56) Entgegenhaltungen:
- WO-A-91/05234
- DE-A- 2 404 871
- US-A- 4 089 209
- LEVERMANN M.: 'TOC-Messung im on-line-Verfahren' CHEMIE, UMWELT, TECHNIK 1994, HEIDELBERG, DE, Seiten 12 - 15, XP000197824

## Beschreibung

Die Erfindung betrifft eine Vorrichtung zur Untersuchung von Flüssigkeitsproben, mit einer Probenkammer, in der mindestens eine Untersuchungseinrichtung angeordnet ist, mit einer Steuer- und Auswerteeinrichtung, und mit einer Befüll- und Entleereinrichtung, durch die jeweils eine Flüssigkeitsprobe aus einer Flüssigkeitsmenge der Probenkammer zugeführt und aus dieser abgeführt wird.

Ein typisches Einsatzgebiet derartiger Untersuchungsvorrichtungen ist die Abwasseranalyse. Hierbei ist es erforderlich, eine Flüssigkeitsprobe aus dem Abwasser zu entnehmen und in der Probenkammer zu untersuchen, wobei oftmals ein Reagenz in der Probenkammer zugesetzt und eine Reaktion der Flüssigkeitsprobe durchgeführt und vermessen wird. Für Messungen innerhalb der Probenkammer werden üblicherweise gas- oder ionenselektive Sensoren, pH-Sensoren, photooptische Sensoren und andere Sensoren verwendet. Ein bei der Reaktion entstehendes gasförmiges Reaktionsprodukt kann einer hierfür vorgesehenen Meßeinheit zugeführt werden, beispielsweise einem CO₂-Detektor (Zeitschriftenveröffentlichung M. Levermann "TOC-Messung im on-line-Verfahren", Zeitschrift "Chemie, Umwelt, Technik" 94, Seiten 12-15).

Zum Befüllen der Probenkammer muß die Flüssigkeitsprobe aus der zur Verfügung stehenden Flüssigkeitsmenge, beispielsweise aus dem Abwasser, über eine Zufuhrleitung und eine Pumpe in die im Gerät angeordnete Probenkammer gefördert werden. In umgekehrter Richtung erfolgt das Entleeren der Probenkammer ebenfalls über Flüssigkeitsleitungen.

Insbesondere bei der Untersuchung von Abwasserproben ist es unvermeidlich, daß die zum Befüllen und zum Entleeren der Probenkammer notwendigen Flüssigkeitsleitungen verschmutzen. Es besteht die Gefahr, daß sich diese Leitungen zusetzen, wenn keine regelmäßige Spülung erfolgt, die mit verhältnismäßig großem Aufwand verbunden ist.

Aufgabe der Erfindung ist es daher, eine Vorrichtung der eingangs genannten Gattung so auszubilden, daß das Befüllen und Entleeren der Probenkammer in besonders einfacher Weise erfolgen kann, ohne daß die Gefahr eines Zusetzens von Flüssigkeitsleitungen besteht und ein erheblicher Spülaufwand erforderlich wird.

Diese Aufgabe wird erfindungsgemäß dadurch gelöst, daß die Probenkammer in einer in die Flüssigkeitsmenge eintauchbaren Meßboje angeordnet ist und über eine Kammeröffnung mit der Außenseite der Meßboje in Verbindung steht, daß mindestens eine Untersuchungseinrichtung in der Meßboje angeordnet ist, und daß die Befüll- und Entleereinrichtung eine Gaswechseleinrichtung aufweist, mit der ein die Flüssigkeitsprobe verdrängendes Gas in die Probenkammer eingeführt und daraus abgeführt wird.

Durch die Verlagerung der Probenkammer aus einem außerhalb der Flüssigkeit angeordneten Meßgerät in eine in die Flüssigkeitsmenge eintauchende Meßboje entfällt die Notwendigkeit von zur Verschmutzung neigenden Flüssigkeitsleitungen. Die innerhalb der zu untersuchenden Flüssigkeit angeordnete Probenkammer kann auf direktem Wege befüllt und entleert werden, ohne daß hierfür irgendwelche Flüssigkeitsleitungen erforderlich wären.

Da zumindest der die Probenkammer enthaltende Abschnitt der Meßboje in die zu untersuchende Flüssigkeit eintaucht, reicht es zum Befüllen der Probenkammer aus, das in der Probenkammer enthaltene Gas abzuführen, d.h. im einfachsten Fall die Probenkammer zur Atmosphäre zu öffnen. Der hydrostatische Druck der die Meßboje umgebenden Flüssigkeit drückt die Flüssigkeitsprobe in die Probenkammer. Zum Entleeren der Probenkammer wird durch die Gaswechseleinrichtung Gas, beispielsweise Luft, unter Druck zugeführt, um die Flüssigkeitsprobe aus der Probenkammer herauszudrücken. Die Intensität der Gaszuführung kann dabei so gewählt werden, daß es zu einer turbulenten Verwirbelung der Flüssigkeitsprobe in der Probenkammer kommt, wodurch in sehr einfacher Weise eine wirksame Spülung und Reinigung der Probenkammer und der Kammeröffnung erfolgt. Dadurch ist eine Verstopfung der Kammeröffnung weitestgehend ausgeschlossen.

Die Steuer- und Auswerteeinrichtung kann entfernt von der Meßboje angeordnet und mit dieser über Leitungen verbunden sein. Über Leitungen, die die Meßboje mit der entfernt hierzu angeordneten Steuer- und Auswerteeinrichtung verbinden, werden - neben elektrischen Meßsignalen und ggf. elektrischen Steuerimpulsen oder elektrischer Antriebsenergie - allenfalls Reagenzen und/oder Gase transportiert. Der Transport dieser Stoffe ist im Vergleich zum Transport von Flüssigkeitsproben völlig problemlos und führt nicht zur Gefahr von Verunreinigungen oder Verstopfungen. Stattdessen kann die Steuer- und Auswerteeinrichtung auch in der Meßboje angeordnet sein.

Vorzugsweise ist in der Meßboje mindestens eine in die Probenkammer mündende Reagenz-Dosiereinrichtung angeordnet, die mit einer außerhalb der Meßboje angeordneten Reagenzquelle über eine Schlauchleitung verbunden ist. Dadurch können in der Probenkammer auch solche Untersuchungen durchgeführt werden - die wie dies inbesondere bei der Abwasseruntersuchung häufig der Fall ist - eine chemische Reaktion der Flüssigkeitsprobe mit einem oder mehreren Reagenzen erforderlich machen. Für die Transportleitungen für die flüssigen oder gasförmigen Reagenzen besteht keine Verstopfungsgefahr; sie können deshalb auch über verhältnismäßig große Entfernungen zwischen der Meßboje und einer Versorgungseinheit geführt werden.

Bevorzugt weist die Gaswechseleinrichtung eine in der Meßboje angeordnete, an die Probenkammer angeschlossene Gaspumpe auf, die mit einer außerhalb der Meßboje angeordneten Gasquelle über eine Schlauchleitung verbunden sein kann. Eine solche Schlauchleitung unterliegt ebenfalls keiner Verstopfungsgefahr und kann deshalb problemlos über eine größere Entfernung verlegt werden.

Gemäß einer bevorzugten Ausführungsform der Erfindung ist vorgesehen, daß die Kammeröffnung der Probenkammer in eine darunter in der Meßboje angeordnete Absetzkammer mündet, die eine Bodenöffnung aufweist. Vor allem bei der Untersuchung des wässrigen Anteils von Belebtschlamm einer Kläranlage ist es notwendig, den Feststoffanteil vor der Untersuchung abzutrennen. Die der Probenkammer vorgeschaltete Absetzkammer dient dazu, die Flüssigkeitsprobe während einer Befüllpause aufzunehmen, so daß sich der Belebtschlammanteil im unteren Bereich der Absetzkammer absetzt bzw. konzentriert, bevor die auf diese Weise vorgereinigte Flüssigkeitsprobe in die Probenkammer eingelassen wird.

Um diesen zeitlichen Ablauf der Befüllung der Probenkammer zu steuern, ist vorzugsweise im Bereich der die Absetzkammer mit der Probenkammer verbindenden Kammeröffnung ein Füllstandssensor angeordnet, der mit der Steuerung für die Gaswechseleinrichtung verbunden ist. Durch den Füllstandssensor wird festgestellt, wann die Absetzkammer gefüllt ist. In diesem Zustand wird der Befüllvorgang unterbrochen, damit sich der Schlammanteil in der Absetzkammer absetzen kann. Nach dieser vorgegebenen Zeitspanne wird der Befüllvorgang fortgesetzt.

Für die Untersuchung von Belebtschlamm, in dem ständig Gasblasen aufsteigen, hat es sich als zweckmäßig erwiesen, im Abstand unter der Bodenöffnung der Absetzkammer einen in der lotrechten Projektion allseitig über die Bodenöffnung hinausreichenden Abweiskörper anzuordnen. Dieser Abweiskörper verhindert, daß Gasblasen in die Absetzkammer und in die Probenkammer eintreten.

Gemäß einer anderen vorteilhaften Ausführungsform der Erfindung ist vorgesehen, daß die Probenkammer mit einer Gaszufuhrleitung für ein Reaktionsgas verbunden ist, daß die Kammeröffnung durch ein Ventil verschließbar ist und daß eine durch ein Ventil verschließbare Gasabführleitung aus der Probenkammer zu einer entfernt von der Meßboje angeordneten Auswerteeinrichtung führt. Dadurch ist es möglich, die in der Probenkammer aufgenommene Flüssigkeitsprobe nach dem Verschließen der Kammeröffnung einer Reaktion mit dem Reaktionsgas zu unterwerfen und dieses Reaktionsgas danach aus der Meßboje heraus zu der entfernt angeordneten Auswerteeinrichtung zu leiten, um dort die erforderliche Analyse durchzuführen.

Weitere vorteilhafte Ausgestaltungen der Erfindung sind Gegensstand weiterer Unteransprüche.

Nachfolgend werden Ausführungsbeispiele der Erfindung näher erläutert, die in der Zeichnung dargestellt sind. Es zeigt:
Fig. 1 in einem senkrechten Schnitt eine Vorrichtung zur Bestimmung des NH4-Gehalts von Abwasser,
Fig. 2 einen Schnitt längs der Linie II-II in Fig. 1,
Fig. 3 und 4 in einer Darstellung entsprechend den Fig. 1 und 2 eine Vorrichtung zur Bestimmung des Nitrat- bzw. Phosphatgehalts einer Abwasserprobe,
Fig. 5 und 6 in Darstellungen entsprechend den Fig. 1 und 2 eine Vorrichtung zur Bestimmung des Nitratgehalts von Abwasser,
Fig.7 und 8 in Darstellungen entsprechend den Fig. 1 und 2 eine Vorrichtung zur Bestimmung des TOC-Gehalts von Abwasser,
Fig.9 in einem vereinfachten senkrechten Schnitt eine Vorrichtung zur Bestimmung des biologischen Sauerstoffbedarfs (BSB) einer Abwasserprobe,
Fig. 10 in einem vereinfachten senkrechten Schnitt eine andere Ausführungsform einer Vorrichtung zur Bestimmung des BSB einer Abwasserprobe und
Fig. 11 eine Vorrichtung zur Bestimmung des chemischen Sauerstoffbedarfs (CSB) einer Abwasserprobe, ebenfalls in einem vereinfachten senkrechten Schnitt ähnlich den vorangehenden Figuren.

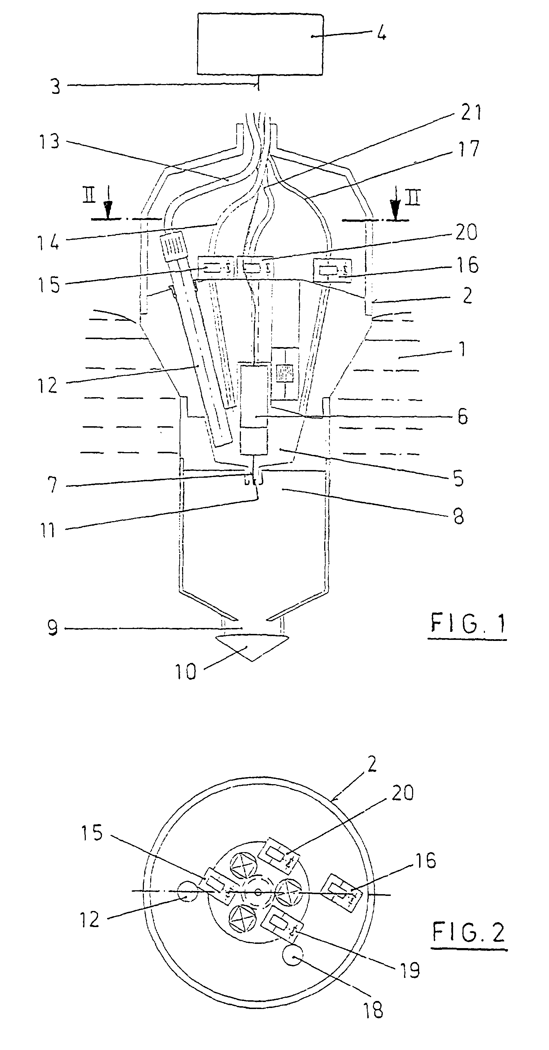

Die in den Fig. 1 und 2 dargestellte Vorrichtung zur Bestimmung des NH₄-Gehalts von Wasser weist eine in das zu untersuchende Abwasser 1 eintauchbare Meßboje 2 auf, die über nur schematisch angedeutete Leitungen 3 mit einer entfernt davon angeordneten Steuer- und Auswerteeinrichtung 4 verbunden ist. In der Meßboje 2 ist eine Probenkammer 5 angeordnet, die die zu untersuchende Abwasserprobe aufnimmt. In die Probenkammer 5 ragt ein Rührwerk 6. Die Probenkammer 5 weist am Boden eine Kammeröffnung 7 auf, durch die die Probenkammer 5 befüllt und entleert werden kann. Unterhalb der Kammeröffnung 7 ist in der Meßboje 2 eine Absetzkammer 8 angeordnet, deren Volumen größer als das der Probenkammer 5 ist. Die Absetzkammer 8 weist eine Bodenöffnung 9 auf, unter der ein Abweiskörper 10 im Abstand angeordnet ist. Der Abweiskörper 10 ragt in der lotrechten Projektion allseitig über die Bodenöffnung 9 hinaus und bewirkt, daß die bei einer Belebtschlammuntersuchung aufsteigenden Gasblasen nicht durch die Bodenöffnung 9 in die Absetzkammer 8 eintreten können. Im Bereich der die Absetzkammer 8 mit der Probenkammer 5 verbindenden Kammeröffnung 7 ist ein Füllstandssensor 11 angeordnet.

Bei dem in den Fig. 1 und 2 dargestellten Ausführungsbeispiel ragt in die Probenkammer 5 eine NH3-Sonde 12, die über eine Leitung 13 mit der Steuer- und Auswerteeinrichtung 4 verbunden ist. Eine Schlauchleitung 14 führt von einer außerhalb der Meßboje 2 angeordneten (nicht dargestellten) Reagenzquelle zu einem Magnetventil 15, das eine Reagenz-Dosiereinrichtung zur Zufuhr eines Reagenz in die Probenkammer 5 bildet. In entsprechender Weise dient eine mit einem Magnetventil 16 verschließbare Schlauchleitung 17 zur Zufuhr von Kalibrierstandard zur Probenkammer 5.

Eine ebenfalls in die Probenkammer 5 ragende pH-Sonde 18 dient zur Bestimmung des pH-Werts in der Flüssigkeitsprobe. Eine Luftleitung, in der ein Magnetventil 19 angeordnet ist, mündet ebenfalls in die Probenkammer 5.

Zur Untersuchung einer Abwasserprobe werden die Probenkammer 5 und die Absetzkammer 8 durch Zufuhr von Luft oder Gas durch Öffnen des Magnetventils 19 entleert. Nach Öffnen eines Magnetventils 20, das ebenfalls an die Probenkammer 5 angeschlossen ist, entweicht die in der Probenkammer 5 und der Absetzkammer 8 enthaltene Luft über eine Schlauchleitung 17, und die Absetzkammer 8 wird gefüllt, bis der Füllstandssensor 11 anspricht. Nach einer Absetzpause, in der sich der in der Flüssigkeitsprobe enthaltene Schlammanteil und ggf. weitere absetzbare Stoffe in der Absetzkammer 8 nach unten abgesetzt haben, erfolgt durch erneutes Öffnen des Magnetventils 16 die Befüllung der Probenkammer 5 unter der Wirkung des hydrostatischen Drucks des umgebenden Abwassers.

Sodann wird durch Öffnen des Magnetventils 15 Lauge in die Probenkammer 5 eingelassen, bis sich an der Sonde 18 ein pH-Wert von etwa 11 eingestellt hat. Bei einem pH-Wert von 11 liegt das gesamte NH₄ nach ausreichender Durchmischung als NH₃ vor und wird mit der NH₃-Sonde 12 vermessen und über einen Rechner in der Steuer- und Auswerteeinrichtung 4 ausgewertet. Danach kann ein erneuter Meßzyklus in der beschriebenen Weise durchgeführt werden.

Zur Kalibrierung der Vorrichtung kann eine Standardleitung 17 über ein Magnetventil 16 an die Probenkammer 5 angeschlossen sein, um eine Standardflüssigkeit zuzuführen, so daß eine automatische Kalibrierung vorgenommen werden kann.

In ähnlicher Weise kann eine (nicht dargestellte) NO₃-Meßvorrichtung ausgeführt sein. Anstelle der beschriebenen NH₃-Sonde 12 ist dann eine NO₃-Sonde vorgesehen. Außerdem wird eine zusätzliche Leitfähigkeitssonde in der Probenkammer 5 angebracht.

Bei allen folgenden Ausführungsbeispielen sind gleiche Teile mit gleichen Bezugszeichen wie bei den Fig. 1 und 2 bezeichnet.

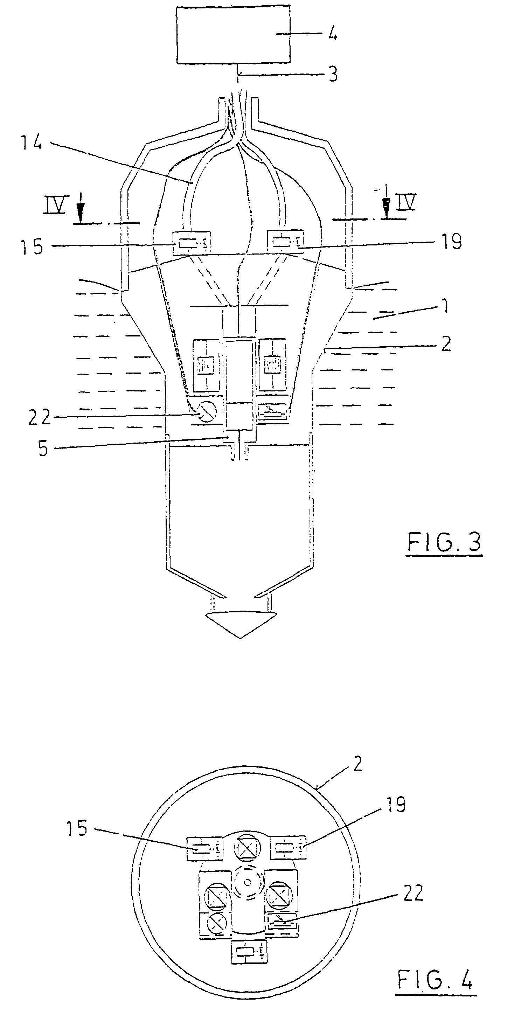

Die Fig. 3 und 4 zeigen als Ausführungsbeispiel der Erfindung eine Vorrichtung zur Bestimmung des Nitrat-Phosphatgehalts von Abwasser. An der Probenkammer 5 ist eine optische Meßzelle 22 angeordnet, die ein Signal an die Steuer- und Auswerteeinrichtung 4 liefert, wenn in der Flüssigkeitsprobe ein Farbumschlag auftritt, der durch die dosierte Zufuhr von Reagenz durch die Schlauchleitung 14 und das Magnetventil 15 auftritt.

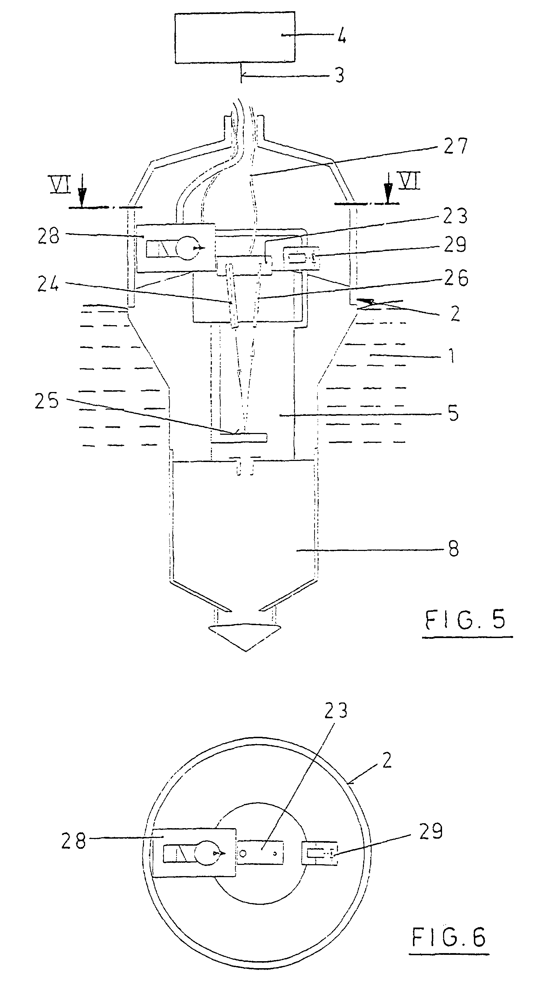

Die Fig. 5 und 6 zeigen eine Vorrichtung zur Bestimmmung des Nitratgehalts von Abwasser mittels eines optischen Meßgeräts 23, das in der Meßboje 2 angeordnet ist. Ein Lichtwellensender 24 wirft einen Lichtstrahl auf einen Reflektor 25 in der Probenkammer 5, der von dort reflektiert wird und auf einen Lichtwellenempfänger 26 trifft. Die elektrische Antriebsenergie für die optische Einrichtung 23 und die Signalübermittlung zur Steuer- und Auswerteeinrichtung 4 erfolgt über elektrische Leitungen 27. Als Gaswechseleinrichtung zum Befüllen und Entleeren der Probenkammer 5 und der Absetzkammer 8 dient ein Kompressor 28, der über ein Magnetventil 29 an die Probenkammer 5 angeschlossen ist. Nach dem Entleeren der Probenkammer 5 kann eine Referenzmessung durchgeführt werden, um die durch Verschmutzungen am Lichtwellensender 24, am Reflektor 25 und/oder am Lichtwellenempfänger 26 auftretenden Signalveränderungen zu kompensieren.

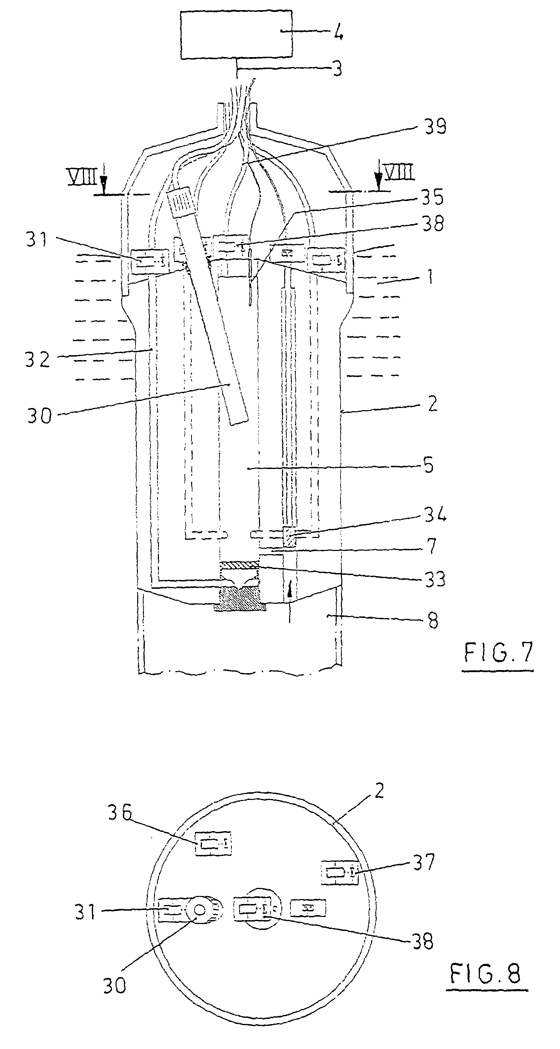

Das in den Fig. 7 und 8 gezeigte Ausführungsbeispiel der Erfindung stellt eine Vorrichtung zur Untersuchung des oxidierbaren Kohlenstoffgehalts (TOC-Gehalt) von Abwasser dar. Die Meßsonde 2 wird auch hierbei in das Abwasser 1 eingetaucht. In die Probenkammer 5 ragt eine pH-Sonde 30.

Eine mit einem Magnetventil 31 verschließbare Gaszufuhrleitung 32 mündet an Boden der Probenkammer 5 unter einer Fritte 33, um Reaktionsgas (O₃) in die Probenkammer 5 zu leiten. Um zu verhindern, daß das in die Probenkammer 5 eingebrachte Reaktionsgas die Probenkammer 5 entleert, ist im Bereich der Kammeröffnung 7 ein Ventil 34 angeordnet, das geschlossen wird, wenn Reaktionsgas der Probenkammer 5 zugeführt wird.

Ein in die Probenkammer 5 ragender Füllstandssensor 35 gibt ein Signal, wenn die Probenkammer 5 vollständig gefüllt ist. Über Magnetventile 36 bzw. 37 können Säure bzw. Lauge der Probenkammer 5 zugeführt werden. Nach der Reaktion mit der in der Probenkammer 5 enthaltenen Abwasserprobe gelangt das Reaktionsgas über ein Magnetventil 38 und eine Gasabführleitung 39 zu einer entfernt von der Meßboje 2 in der Steuer- und Auswerteeinrichtung 4 angeordneten Auswerteeinrichtung. Die dort durchgeführte Analyse des austretenden Reaktionsgases liefert einen Meßwert für den TOC-Gehalt des untersuchten Abwassers.

In Fig. 7 ist im unteren Bereich der Meßboje 2 eine Absetzkammer 8 mit strichpunktierten Linien nur angedeutet. Damit soll dargestellt werden, daß auf eine solche Absetzkammer 8 verzichtet werden kann. Insbesondere bei der TOC-Bestimmung kann es erforderlich sein, auf das Absetzen von Feststoffanteilen in dem zu untersuchenden Abwasser zu verzichten, wenn diese Feststoffanteile bei der Bestimmung des TOC-Gehalts berücksichtigt werden müssen.

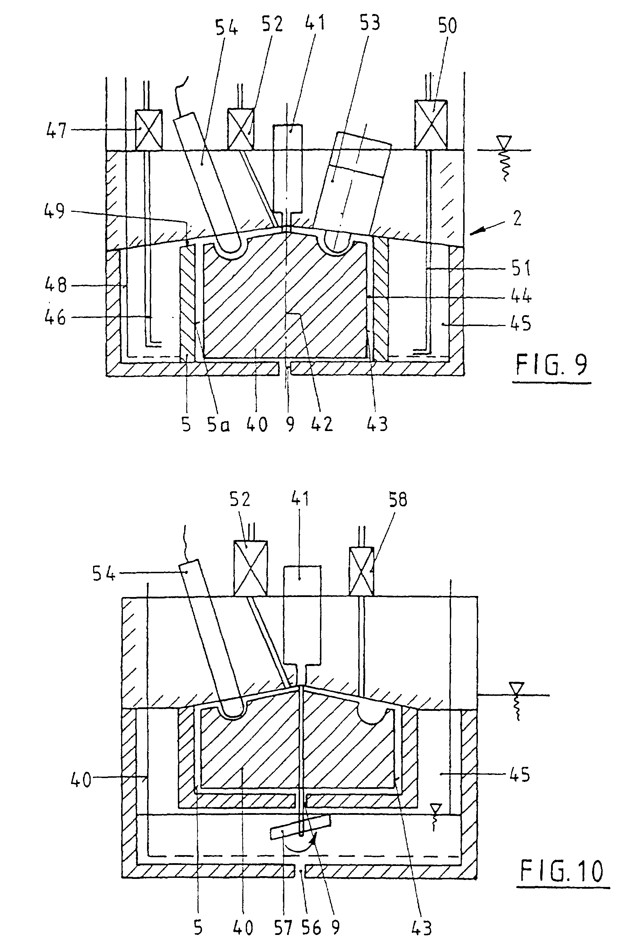

Die in Fig. 9 gezeigte Vorrichtung dient zur Bestimmung des biologischen Sauerstoffbedarfs (BSB) von Abwasser. Die Vorrichtung ist als eine in das Abwasser absenkbare Meßboje 2 ausgeführt. In der Probenkammer 5 ist ein antriebbarer Rotor 40 angeordnet, der durch einen Motor 41 zu Drehungen um die senkrechte Achse 42 angetrieben wird. Die Außenfläche 43 des Rotors 40 bildet eine biologische Aufwuchsfläche. Zwischen der Außenfläche 43 des Rotors 40 und der Wand 5a der Probenkammer 5 besteht nur ein schmaler Spalt 44, der die Reaktionskammer bildet.

Die Probenkammer 5 steht über die im Boden angeordnete Kammeröffnung 9 unmittelbar mit der umgebenden Flüssigkeit in Verbindung. Die Probenkammer 5 ist von einer Belüftungskammer 45 umgeben, die zum Belüften und Temperieren des verwendeten Verdünnungswassers dient. Zu diesem Zweck ragen ein Belüfter 46, der mit einemr Zuluftventile 47 verbunden ist, und einer Heizung 48 in die Belüftungskammer 45. Die Belüftungskammer 45 mündet über eine Überlauföffnung 49 in die Probenkammer 5. Über eine Zufuhreinrichtung 50, die als Pumpe oder als Ventil ausgeführt sein kann, wird der Belüftungskammer 45 durch eine Leitung 51 Verdünnungswasser zugeführt.

Nach Beendigung einer vorangegangenen Messung wird das temperierte Verdünnungswasser in der Belüftungskammer 45 belüftet. Die Luft verläßt die Meßboje 2 dabei über die Überlauföffnung 49, den oberen Bereich der Probenkammer 5 und über ein Luftauslaßventil 52.

Nach ausreichender Belüftung wird das Zuluftventil 47 geschlossen und das Ventil 50 für den Wasserzulauf geöffnet. Sobald durch einen Kontaktgeber ein ausreichender Füllstand festgestellt wird, wird das Luftauslaßventil 52 geschlossen.

Das temperierte, belüftete Verdünnungswasser verdrängt jetzt das Abwassergemisch der vorangegangenen Messung in der Probenkammer 5, das durch die Kammeröffnung 9 austritt. Nach Austausch des Abwassergemischs in der Probenkammer 5 durch Verdünnungswasser beginnt einer erneute Messung. Durch eine Dosierpumpe 53 wird dann Abwasser in die Probenkammer 5 gesaugt. Eine Sauerstoffsonde 54 dient zur Bestimmung des Sauerstoffverbrauchs pro Zeiteinheit und damit zur Bestimmung des BSB.

Durch die Verwendung des Rotors 40 als Träger der biologischen Aufwuchsfläche wird erreicht, daß diese Aufwuchsfläche in innige Berührung mit der gesamten, in der Probenkammer 5 enthaltenen Probe gelangt. Als besonders vorteilhaft ist dabei anzusehen, daß das Volumen der Probenkammer 5 sehr gering ist.

Die in Fig. 10 gezeigte Vorrichtung zur Bestimmung des BSB unterscheidet sich von der vorher beschriebenen Vorrichtung im wesentlichen dadurch, daß sie ohne Verdünnungswasser arbeitet. Auch hierbei ist ein durch den Motor 41 antreibbarer Rotor 40 in der Probenkammer 5 angeordnet. Seine Außenfläche 43 bildet die biologische Aufwuchsfläche. Die Kammeröffnung 9 am Boden der Probenkammer 5 mündet in eine Beiüftungskammer 55, die die Probenkammer 5 am Umfang und am Boden umgibt. Diese Belüftungskammer 55 weist am Boden eine Durchtrittsöffnung 56 für das Abwasser auf. Der Rotor 40 ist mit einem in die Belüftungskammer 55 ragenden Belüfter 57 verbunden. Eine mit einem Zuluftventil 58 verbundene Leitung mündet in die Probenkammer 5. Im übrigen sind gleiche Teile wie beim Beispiel nach Fig. 9 mit gleichen Bezugszeichen versehen.

Die Probenkammer 5, die die Reaktionskammer bildet, und die Belüftungskammer 55 werden durch über das Ventil 58 einströmende Luft entleert. Dann wird Abwasser durch den hydrostatischen Druck des umgebenden Abwassers durch die Durchtrittsöffnung 56 in die Belüftungskammer 55 gedrückt, dort belüftet und temperiert. Das belüftete Abwasser wird sodann in die Probenkammer 5 durch die Kammeröffnung 9 gedrückt. Mittels der Sauerstoffsonde 54 wird die Sauerstoffzehrung pro Einheit gemesssen und in den biologischen Sauerstoffbedarf (BSB) umgerechnet.

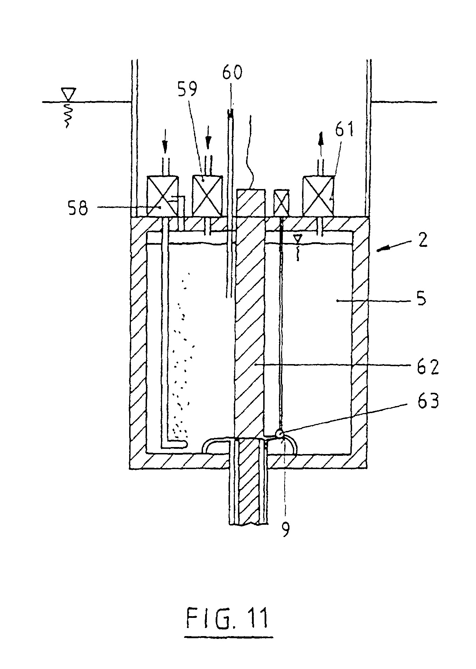

Fig. 11 zeigt eine als eintauchbare Meßboje ausgeführte Vorrichtung zur Bestimmung des chemischen Sauerstoffbedarfs (CSB). In die Probenkammer 5 mündet eine durch ein Ventil 58 verschließbare Leitung für die Zufuhr von Luft oder Ozon. Über ein Reagenzventil 59 können Reagenzien zugeführt werden. In die Probenkammer 5 ragt eine pH-Sonde 60. Durch ein Auslaßventil 61 können Luft oder Ozon aus der Probenkammer 5 abgelassen werden. Eine Ozonmeßsonde 62 dient der Bestimmung des Ozongehalts. Die Kammeröffnung 9 der Probenkammer 5 ist durch ein Ventil 63 verschließbar.

Bei geöffnetem Einlaßventil 58 und geöffnetem Auslaßventil 61 wird die Probenkammer 5 gefüllt. Der pH-Wert wird auf einem vorgegebenen Wert im Bereich von 3 bis 5 eingestellt.

Das in der Probenkammer 5 enthaltene Abwasser wird mit Ozon begast. Nach ausreichender Begasung wird der (nicht dargestellte) Ozongenerator abgeschaltet und durch das als Dreiwegeventil ausgeführte Einlaßventil 58 wird nach Umschalten nur Luft oder Sauerstoff in einer vorgegebenen Menge auf die Wasseroberfläche gedrückt. Bei offenem Ventil 63 ist die Ozonzehrung pro Zeiteinheit, die an der Kammeröffnung 9 gemessen wird, ein Maß für den CSB. Nach der Entleerung beginnt die Messung erneut.

## Patentansprüche

1. Vorrichtung zur Untersuchung von Flüssigkeitsproben, mit einer Probenkammer (5), in der mindestens eine Untersuchungseinrichtung angeordnet ist, mit einer Steuer- und Auswerteeinrichtung (4) und mit einer Befüll- und Entleereinrichtung, durch die jeweils eine Flüssigkeitsprobe aus einer Flüssigkeitsmenge der Probenkammer (5) zugeführt und aus dieser abgeführt wird, **dadurch gekennzeichnet, daß** die Probenkammer (5) in einer in die Flüssigkeitsmenge (1) eintauchbaren Meßboje (2) angeordnet ist und über eine Kammeröffnung (7) mit der Außenseite der Meßboje (2) in Verbindung steht, daß mindestens eine Untersuchungseinrichtung in der Meßboje (2) angeordnet ist, und daß die Befüll- und Entleereinrichtung eine Gaswechseleinrichtung aufweist, mit der ein die Flüssigkeitsprobe verdrängendes Gas in die Probenkammer (5) eingeführt und daraus abgeführt wird.

2. Vorrichtung nach Anspruch 1, **dadurch gekennzeichnet, daß** die Steuer und Auswerteeinrichtung (4) entfernt von der Meßboje (2) angeordnet und mit dieser über Leitungen (3) verbunden ist.

3. Vorrichtung nach Anspruch 1, **dadurch gekennzeichnet, daß** die Steuer- und Auswerteeinrichtung (4) in der Meßboje angeordnet ist.

4. Vorrichtung nach Anspruch 1, **dadurch gekennzeichnet, daß** in der Meßboje (2) mindestens eine in die Probenkammer (5) mündende Reagenz-Dosiereinrichtung (15) angeordnet ist, die mit einer außerhalb der Meßboje (2) angeordneten Reagenzquelle über eine Schlauchleitung (14) verbunden ist.

5. Vorrichtung nach Anspruch 1, **dadurch gekennzeichnet**, die Gaswechseleinrichtung eine in der Meßboje (2) angeordnete, an die Probenkammer (5) angeschlossene Gaspumpe (28) aufweist.

6. Vorrichtung nach Anspruch 1, **dadurch gekennzeichnet, daß** die Kammeröffnung (7) in eine darunter in der Meßboje (2) angeordnete Absetzkammer (8) mündet, die eine Bodenöffnung (9) aufweist.

7. Vorrichtung nach Anspruch 6, **dadurch gekennzeichnet, daß** im Bereich der die Absetzkammer (8) mit der Probenkammer (5) verbindenden Kammeröffnung (7) ein Füllstandssensor (11) angeordnet ist, der mit der Steuerung für die Gaswechseleinrichtung verbunden ist.

8. Vorrichtung nach Anspruch 6, **dadurch gekennzeichnet, daß** im Abstand unter der Bodenöffnung (9) ein in der lotrechten Projektion allseitig über die Bodenöffnung (9) hinausreichender Abweiskörper (10) angeordnet ist.

9. Vorrichtung nach Anspruch 2, **dadurch gekennzeichnet, daß** die Probenkammer (5) mit einer Gaszufuhrleitung (32) für ein Reaktionsgas verbunden ist, daß die Kammeröffnung (7) durch ein Ventil (34) verschließbar ist, und daß eine durch ein Ventil (38) verschließbare Gasabführleitung (39) aus der Probenkammer (5) zu einer entfernt von der Meßboje (2) angeordneten Auswerteeinrichtung führt.

10. Vorrichtung nach Anspruch 1, **dadurch gekennzeichnet, daß** in der Probenkammer (5) ein antreibbarer Rotor (40) angeordnet ist, dessen Außenfläche (43) eine biologische Aufwuchsfläche bildet.

11. Vorrichtung nach Anspruch 10, **dadurch gekennzeichnet, daß** zwischen der Außenfläche des Rotors (40) und der Wand (5a) der Probenkammer (5) ein schmaler Spalt (44) besteht.

12. Vorrichtung nach Anspruch 10, **dadurch gekennzeichnet, daß** die Probenkammer (5) über die Kammeröffnung (9) unmittelbar mit der umgebenden Flüssigkeit in Verbindung steht und von einer Belüftungskammer (45) für Verdünnungswasser umgeben ist, die über eine Überlauföffnung (49) in die Probenkammer (5) mündet.

13. Vorrichtung nach Anspruch 10, **dadurch gekennzeichnet, daß** die Kammeröffnung (9) der Probenkammer (5) in eine Belüftungskammer (55) mündet, die eine Durchtrittsöffnung (56) für das Abwasser aufweist.

14. Vorrichtung nach Anspruch 1, **dadurch gekennzeichnet, daß** die Kammeröffnung (9) der Probenkammer (5) durch ein Ventil (63) verschließbar ist.

## Claims

1. Device for analysing fluid samples consisting of a sample chamber (5) containing at least one testing device, which comprises a control and analysis device (4) and a filling and draining device, through which, respectively, a fluid sample taken from a quantity of fluid is fed into the sample chamber (5) and removed from it, **characterised in that** the sample chamber (5) is arranged in a measuring buoy (2) immersible in a quantity of fluid (1) and is connected to the outside of the measuring buoy (2) via a chamber opening (7), that at least one testing device is arranged in the measuring buoy (2), and that the filling and draining device comprises a gas exchange apparatus with which a gas which displaces the fluid sample is fed into and removed from the sample chamber (5).

2. Device according to claim 1, **characterised in that** the control and analysis device (4) is arranged at a distance from the measuring buoy (2) and is connected to it by lines (3).

3. Device according to claim 1, **characterised In that** the control and analysis device (4) is arranged in the measuring buoy.

4. Device according to claim 1, **characterised in that** at least one reagent dosing device (15), which opens into the sample chamber (5) and Is connected to a reagent source located outside the measuring buoy (2) via a hose assembly (14), is arranged in the measuring buoy (2).

5. Device according to claim 1, **characterised in that** the gas exchange device comprises a gas pump (28) arranged in the measuring buoy (2) and connected to the sample chamber (5).

6. Device according to claim 1, **characterised in that** the chamber opening (7) opens into a settling chamber (8) with a base opening (9), which is arranged underneath it in the measuring buoy (2).

7. Device according to claim 6, **characterised in that** a fill level sensor (11), which is connected to the control unit for the gas exchange device, is positioned near the chamber opening (7) connecting the settling chamber (8) to the sample chamber (5).

8. Device according to claim 6, **characterised in that** a deflection body (10), which in perpendicular projection extends beyond the base opening (9) on all sides, is positioned at a distance beneath the base opening (9).

9. Device according to claim 2, **characterised in that** the sample chamber (5) is linked to a gas supply line (32) for a reaction gas, that the chamber opening (7) can be closed by means of a valve (34), and that a gas discharge line (39) which can be closed by means of a valve (38) runs from the sample chamber (5) to an analysis device located at a distance from the measuring buoy (2).

10. Device according to claim 1, **characterised in that** a drivable rotor (40) is arranged in the sample chamber (5), the outer surface (43) of which forms a biological growth surface.

11. Device according to claim 10, **characterised in that** there is a narrow gap (44) between the outer surface of the rotor (40) and the wall (5a) of the sample chamber (5).

12. Device according to claim 10, **characterised in that** the sample chamber (5) is in direct contact with the surrounding fluid via the chamber opening (9) and is surrounded by an aeration chamber (45) for dilution water which opens into the sample chamber (5) through an overflow opening (49).

13. Device according to claim 10, **characterised in that** the chamber opening (9) of the sample chamber (5) opens into an aeration chamber (55), which comprises an opening (56) for the waste water.

14. Device according to claim 1, **characterised in that** the chamber opening (9) of the sample chamber (5) can be closed by means of a valve (63).

## Revendications

1. Dispositif pour l'analyse d'échantillons de liquides présentant une chambre d'échantillons (5) dans laquelle est disposé au moins un équipement d'analyse, un équipement de commande et d'évaluation (4) et un équipement de remplissage et de vidange par lequel un échantillon de liquide provenant d'une quantité de liquide est à chaque fois amené à la chambre d'échantillons (5) et évacué de celle-ci, **caractérisé en ce que** la chambre d'échantillons (5) est disposée dans une bouée de mesure (2) pouvant être immergée dans la quantité de liquide (1) et est en liaison via une ouverture de chambre (7) avec le côté extérieur de la bouée de mesure (2) **en ce qu'**au moins un équipement d'analyse est disposé dans la bouée de mesure (2) et **en ce que** l'équipement de remplissage et de vidange présente un équipement de remplacement de gaz au moyen duquel un gaz déplaçant l'échantillon de liquide est introduit dans la chambre d'échantillons (5) et est évacué de celle-ci.

2. Dispositif selon la revendication 1, **caractérisé en ce que** l'équipement de commande et d'évacuation (4) est disposé à distance de la bouée de mesure (2) et est connecté avec celle-ci via des fils (3).

3. Dispositif selon la revendication 1, **caractérisé en ce que** l'équipement de commande et d'évaluation (4) est disposé dans la bouée de mesure.

4. Dispositif selon la revendication 1, **caractérisé en ce qu'**au moins un équipement de dosage de réactif (15) débouchant dans la chambre d'échantillons (5) et raccordé à une source de réactif en dehors de la bouée de mesure (2) via un tuyau flexible (14) est disposé dans la bouée de mesure (2).

5. Dispositif selon la revendication 1, **caractérisé en ce que** l'équipement de remplacement de gaz présente une pompe à gaz (28) disposée dans la bouée de mesure (2) et raccordée à la chambre d'échantillons (5).

6. Dispositif selon la revendication 1, **caractérisé en ce que** l'ouverture de chambre (7) débouche dans une chambre de décantation (8) qui est disposée en-dessous de ladite ouverture dans la bouée de mesure (2) et présente une ouverture dans le fond (9).

7. Dispositif selon la revendication 6, **caractérisé en ce qu'**un détecteur de niveau (11) qui est connecté à la commande pour l'équipement de remplacement de gaz, est disposé dans la zone de l'ouverture de chambre (7) raccordant la zone de décantation (8) à la chambre d'échantillons (5).

8. Dispositif selon la revendication 6, **caractérisé en ce qu'**un corps de rejet (10) dépassant l'ouverture dans le fond (9) de tous côtés en projection orthogonale est disposé à une distance au-dessous de l'ouverture dans le fond (9).

9. Dispositif selon la revendication 2 **caractérisé en ce que** la chambre d'échantillons (5) est raccordée à une conduite d'alimentation en gaz (32) pour un gaz de réaction, **en ce que** l'ouverture de chambre (7) peut être obturée par une soupape (34), et **en ce qu'**une conduite d'évacuation de gaz (39) obturable par une soupape (38) mène depuis la chambre d'échantillons (5) à un équipement d'évacuation disposé à distance de la bouée de mesure (2).

10. Dispositif selon la revendication 1, **caractérisé en ce qu'**un rotor (40) pouvant être entraîné et dont la surface extérieure (43) forme une surface de croissance biologique est disposé dans la chambre d'échantillons (5).

11. Dispositif selon la revendication 10, **caractérisé en ce qu'**il y a une fente étroite (44) entre la surface extérieure du rotor (40) et la paroi (5a) de la chambre d'échantillons (5).

12. Dispositif selon la revendication 10, **caractérisé en ce que** la chambre d'échantillons (5) est en liaison directe avec le liquide environnant via l'ouverture de chambre (9) et est entourée d'une chambre d'aération (45) pour de l'eau de dilution, qui débouche via une ouverture de trop-plein (49) dans la chambre d'échantillons (5).

13. Dispositif selon la revendication 10, **caractérisé en ce que** l'ouverture de chambre (9) de la chambre d'échantillons (5) débouche dans une chambre d'aération (55) qui présente une ouverture de passage (56) pour l'effluent.

14. Dispositif selon la revendication 1, **caractérisé en ce que** l'ouverture de chambre (9) de la chambre d'échantillons (5) est obturable par une soupape (63).
